# EUROPEAN PATENT APPLICATION

(11) **EP 4 834 895 A1**
(43) Date of publication of application: **04.11.2026**
(21) Application number: 24911434.9
(22) Date of filing: 27.12.2024
(51) Int. Cl.: C07D 401/06, C07D 401/02, A61K 31/454, A61P 37/00, A61P 13/12, C07D 451/06, A61K 31/439, A61P 11/00, A61P 13/00, A61P 27/02

(54) **CRYSTAL OF NITROGEN-BRIDGED HETEROCYCLIC COMPOUND AND PREPRATION METHOD THEREFOR**

(30) Priority: 29.12.2023 CN 202311850886
(71) Applicant: Jiangsu Hengrui Pharmaceuticals Co., Ltd., Lianyungang, Jiangsu 222047 (CN); Suncadia Pharmaceuticals Co., Ltd, Chengdu, Sichuan 610000 (CN); Chengdu Xinyue Medicine Co. Ltd., Chengdu, Sichuan 610000 (CN)
(72) Inventor: ZHAO, Hong, Chengdu, Sichuan 610000 (CN); WAN, Zhenjiang, Chengdu, Sichuan 610000 (CN); XUE, Zhouyang, Chengdu, Sichuan 610000 (CN); ZHANG, Hui, Chengdu, Sichuan 610000 (CN); CHEN, Yu, Chengdu, Sichuan 610000 (CN); HU, Zhipeng, Chengdu, Sichuan 610000 (CN); FAN, Bo, Chengdu, Sichuan 610000 (CN)
(74) Representative: Potter Clarkson
(86) International application number: PCT/CN2024/143079
(87) International publication number: WO 2025/140514

(57) **Abstract**

The present invention relates to a crystal of a nitrogen-bridged heterocyclic compound and a preparation method therefor. Specifically, the present invention relates to a crystal form of a fumarate of a compound represented by formula (I). The crystal provided by the present invention has excellent flowability, and is better for use in production and clinical treatment.

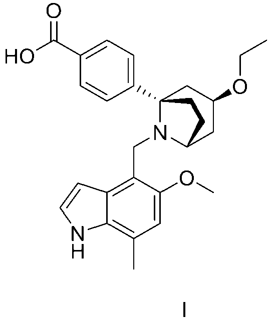

## Description

### TECHNICAL FIELD

The present disclosure pertains to the field of pharmaceutical synthesis and relates to a crystal of a nitrogen-containing bridged heterocyclic compound and a preparation method therefor.

### BACKGROUND

Complement factor B (Factor B) is a thermolabile β globin, which can be inactivated by being simply heated at 50 °C for 30 min. It can be cleaved by complement factor D into two fragments: Ba and Bb, and Bb binds to C3b to form C3 convertase of the alternative pathway. Complement factor B, also known as C3 proactivator, is an important component of the complement alternative activation pathway. Complement factor B has a molecular weight of 93 kDa, is present in human blood at a concentration of about 3 µM, and is synthesized primarily in the liver. It is found that complement factor B is also synthesized in the retinal pigment epithelial cells of the eyes.

Glomerulopathy includes IgA nephropathy (IgAN for short), C3 glomerulopathy (C3G for short), membranous glomerulonephritis (MGN for short), and the like. IgAN and MGN are the most common of them. However, there has been some increase in the incidence of rare kidney diseases such as C3 glomerulopathy over the last decade. Glomerulopathy has been found to be closely related to the complement pathways, especially the complement alternative pathway. Currently, there is a lack of clinically effective treatment regimens for primary glomerulonephritis. Medications such as hormones and immunosuppressants (e.g., cyclophosphamide, mycophenolate mofetil, tacrolimus, cyclosporine A, and the traditional Chinese medicine tripterygium glycosides) are typically used. Other medications include blood-pressure-controlling drugs, diuretics and platelet agglutination inhibitors, anticoagulants, lipid-lowering drugs, Cordyceps formulations, and other kidney-protecting and detoxifying drugs.

IgAN is the most common primary glomerular disease worldwide and pathologically manifests itself in localized mesangial hyperplasia and increases in the matrix accompanied by diffuse mesangial deposition of the IgA protein and often by IgG, C3, and C5b-9 deposition. The complement pathways are therefore thought to correlate with the development and progression of IgAN. Currently, there are two small-molecule drugs targeted at the complement pathways that are undergoing clinical trials. OMS721 is a humanized monoclonal antibody targeting the MASP-2 protein developed by Omeros Inc. The MASP-2 protein is the effector enzyme that activates the lectin pathway of the complement system. At the end of the phase 2 clinical trials of OMS721, the four IgAN patients enrolled in the trial all showed a significantly improved proteinuria index. The drug is currently undergoing phase three clinical trials.

Patent applications that disclose factor B inhibitors include WO2015009616A1, WO2019043609A1, WO2020016749A2, and the like. WO2022143845 provides a series of nitrogen-containing heterocyclic derivatives and performs structural characterization. Biological evaluation is performed on the compound of formula (I), and the results show that the compound exhibits relatively good inhibitory effect on the enzyme activity of Factor B.

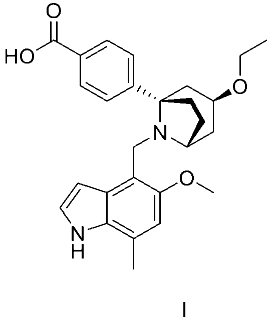

### SUMMARY

The present disclosure is intended to provide a novel crystal of a nitrogen-containing bridged heterocyclic compound and a preparation method therefor. The crystal has good flowability and can be better applied to the manufacture and clinical use of drugs.

In one aspect, the present disclosure provides a crystal of fumarate of a compound of formula (I), wherein the crystal has an angle of repose θ of not greater than 45°; for example, the angle of repose may be not greater than 44°, 43°, 42°, 41°, 40°, 39°, 38°, 37°, 36°, 35°, 34°, 33°, 32°, 31°, or 30°.

In some embodiments, the crystal has a Hausner ratio of not greater than 1.35, e.g., not greater than 1.34, 1.33, 1.32, 1.31, 1.3, 1.29, 1.28, 1.27, 1.26, 1.25, 1.24, 1.23, 1.22, 1.21, or 1.2, e.g., 1-1.3.

In some embodiments, the crystal has a Carr index of not greater than 26%, e.g., not greater than 25%, 24%, 23%, 22%, 21%, 20%, 19%, 18%, 17%, 16%, or 15%.

In some embodiments, the crystal is spherical or ellipsoidal.

In some embodiments, the X-ray powder diffraction pattern of the crystal of the fumarate of the compound of formula (I) described herein, expressed in terms of 2θ angles of diffraction, shows characteristic peaks at 9.6, 14.0, 16.7, 19.6, 25.8, and 26.1.

In some embodiments, the X-ray powder diffraction pattern of the crystal of the fumarate of the compound of formula (I) described herein, expressed in terms of 2θ angles of diffraction, shows characteristic peaks at 6.1, 9.6, 10.0, 14.0, 16.7, 17.2, 19.1, 19.6, 25.8, and 26.1.

In some embodiments, the X-ray powder diffraction pattern of the crystal of the fumarate of the compound of formula (I) described herein, expressed in terms of 2θ angles of diffraction, shows characteristic peaks at 6.1, 9.6, 10.0, 10.8, 14.0, 16.7, 17.2, 18.6, 19.1, 19.6, 25.8, and 26.1.

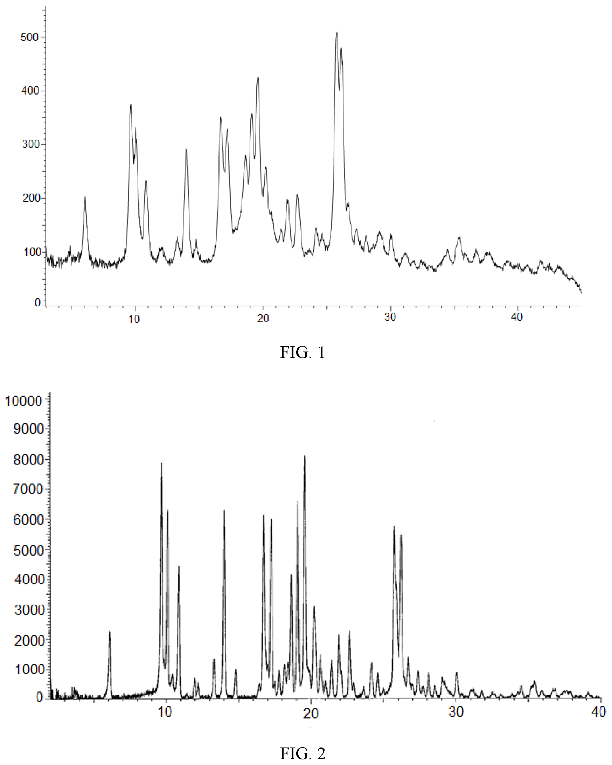

In some embodiments, the X-ray powder diffraction pattern of the crystal of the fumarate of the compound of formula (I) described herein, expressed in terms of 2θ angles of diffraction, is as shown in FIG. 2.

In some embodiments, the fumarate of the compound of formula (I) may be a crystal form I or crystal form II, e.g., crystal form I, of the fumarate of the compound of formula (I). In some embodiments, the content (by weight percentage) of the fumarate of the compound of formula (I) in the crystal of the fumarate of the compound of formula (I) may be greater than 90%, or greater than 92%, or greater than 93%, or greater than 94%, or greater than 95%, or greater than 96%, or greater than 97%, or greater than 98%, or greater than 99%.

In some embodiments, the present disclosure provides a crystal of fumarate of a compound of formula (I), wherein the crystal has an angle of repose θ of not greater than 45°, e.g., not greater than 35°; the crystal has a Hausner ratio of not greater than 1.35, e.g., not greater than 1.26; the crystal has a Carr index of not greater than 26%, e.g., not greater than 21%; for example, the fumarate of the compound of formula (I) is in crystal form I. In some embodiments, the fumarate of the compound of formula (I) has a content (by weight percentage) of greater than 95%, e.g., greater than 97%.

In another aspect, the present disclosure provides a method for preparing the crystal of the fumarate of the compound of formula (I), which comprises: mixing the fumarate of the compound of formula (I) with fumaric acid and a solvent under stirring to precipitate the crystal of the fumarate of the compound of formula (I).

The solvent may be one or more of C₁-C₆ alkyl alcohol (e.g., methanol, ethanol, isopropanol, etc.) and acetone.

In some embodiments, the fumarate of the compound of formula (I) and fumaric acid are first dissolved in a solvent A, followed by mixing with a solvent B to precipitate the crystal of the fumarate of the compound of formula (I), wherein the solvent A may be C₁-C₆ alkyl alcohol, and the solvent B may be acetone.

In some embodiments, the fumarate of the compound of formula (I) and fumaric acid are first dissolved in a solvent A, the solvent is then concentrated, optionally a seed crystal is added, and then a solvent B is added into a resulting system to precipitate the crystal of the fumarate of the compound of formula (I), wherein the solvent A maybe C₁-C₆ alkyl alcohol, and the solvent B may be acetone.

The reaction temperature of the method may be 0-60 °C, such as 0-40 °C.

The present disclosure further relates to a pharmaceutical composition, which comprises the crystal of the fumarate of the compound of formula (I) described herein and one or more pharmaceutically acceptable excipients.

The present disclosure further relates to a pharmaceutical composition, which is prepared by mixing the crystal of the fumarate of the compound of formula (I) described herein with one or more pharmaceutically acceptable excipients.

The present disclosure further relates to a method for preparing a pharmaceutical composition, which comprises mixing the crystal of the fumarate of the compound of formula (I) described herein with one or more pharmaceutically acceptable excipients.

The pharmaceutical composition described herein may be formulated into any one pharmaceutically acceptable dosage form. For example, the crystal or pharmaceutical formulation of the present disclosure may be formulated into a tablet, a capsule, a pill, a crystalline preparation, a solution, a suspension, a syrup, an injection (including a solution for injection, a sterile powder for injection, and a concentrated solution for injection), a suppository, an inhalation, or a spray.

The present disclosure further relates to a drug substance of fumarate of a compound of formula (I), which comprises the crystal of the fumarate of the compound of formula (I) described herein.

The crystal of the fumarate of the compound of formula (I) may have a content of greater than 50%, such as greater than 70%, or greater than 80%, or greater than 90%, based on the total weight of the drug substance.

The present disclosure also provides use of the crystal of the fumarate of the compound of formula (I), the drug substance, or the pharmaceutical composition described above in the manufacture of a medicament for treating a disease or disorder, wherein the disease or disorder is selected from the group consisting of glomerulopathy, hemolytic uremic syndrome, atypical hemolytic uremic syndrome, paroxysmal nocturnal hemoglobinuria, age-related macular degeneration, geographic atrophy, diabetic retinopathy, uveitis, retinitis pigmentosa, macular edema, Behcet's uveitis, multifocal choroiditis, Vogt-Koyanagi-Harada syndrome, birdshot retino-choroiditis, sympathetic ophthalmia, ocular cicatricial pemphigoid, ocular pemphigus, nonarteritic ischemic optic neuropathy, post-operative inflammation, retinal vein occlusion, neurological disorders, multiple sclerosis, stroke, Guillain-Barré syndrome, traumatic brain injury, Parkinson's disease, disorders of inappropriate or undesirable complement activation, hemodialysis complications, hyperacute allograft rejection, xenograft rejection, interleukin-2 induced toxicity during IL-2 therapy, Crohn's disease, adult respiratory distress syndrome, myocarditis, post-ischemic reperfusion conditions, myocardial infarction, balloon angioplasty, post-pump syndrome in cardiopulmonary bypass or renal bypass, atherosclerosis, hemodialysis, renal ischemia, mesenteric artery reperfusion after aortic reconstruction, infectious diseases or sepsis, systemic lupus erythematosus, systemic lupus erythematosus nephritis, proliferative nephritis, liver fibrosis, hemolytic anemia, myasthenia gravis, tissue regeneration, neural regeneration, dyspnea, hemoptysis, acute respiratory distress syndrome, asthma, chronic obstructive pulmonary disease, emphysema, pulmonary embolisms and infarcts, pneumonia, fibrogenic dust diseases, pulmonary fibrosis, asthma, allergy, bronchoconstriction, parasitic disease, Goodpasture's syndrome, pulmonary vasculitis, pauci-immune vasculitis, immune complex-associated inflammation, antiphospholipid syndrome, and obesity; the disease or disorder is preferably C3 glomerulopathy, IgA nephropathy, membranous glomerulonephritis, atypical hemolytic uremic syndrome, and paroxysmal nocturnal hemoglobinuria.

The crystal of the fumarate of the compound of formula (I) prepared in the present disclosure features good flowability, good solubility, rapid dissolution, high crystal form purity, good crystal form stability under light exposure, high temperature, and high humidity conditions, great stability in the preparation, storage, and drying processes, and excellent biocompatibility, and can meet the pharmaceutical requirements for production, transportation, and storage. The production process is stable, repeatable, and controllable, and can be suitable for industrial production.

The crystals obtained by the present disclosure are structurally analyzed and studied using X-ray powder diffraction patterns (XRPD) and differential scanning calorimetry (DSC). The starting materials used in the method for preparing the crystals of the present disclosure may be any form of the fumarate of the compound of formula (I); specific forms include, but are not limited to, an amorphous form, any crystal form, any hydrate or solvate, etc.

As used herein, "X-ray powder diffraction pattern" or "XRPD" refers to a set of X-ray powder diffraction peaks obtained according to the Bragg's equation 2d sin θ = nλ (in the equation, λ is the wavelength of X-ray, λ = 1.54056 Å, the diffraction order n is any positive integer, and the first-order diffraction peak is generally taken, i.e., n = 1). When X-ray is incident on a certain atomic plane with the d-lattice plane spacing of a crystal or a partial crystal sample at a grazing angle θ (the complementary angle of incidence angle, also known as Bragg angle), the Bragg's equation can be satisfied.

As used herein, "differential scanning calorimetry" or "DSC" refers to measurement of the temperature difference and heat flow difference between a sample and a reference substance during a ramping or thermostatic process to characterize all the physical changes and chemical changes related to the thermal effect, and to obtain the phase change information of the sample.

As used herein, "2θ" or "2θ angle" refers to a diffraction angle; θ is the Bragg angle in unit ° or degree, and the 2θ angles have a margin of error of ±0.3, ±0.2, or ±0.1.

As used herein, "interplanar spacing or interplanar spacing (d value)" means that three non-parallel unit vectors (a, b, and c) are selected in the space lattice, each of which connects two adjacent lattice points and all of which divide the lattice into juxtaposed parallelepiped units. The space lattice is divided according to the connecting lines of the determined parallelepiped units, giving a set of linear grids called space lattice or crystal lattice. The lattice and the crystal lattice reflect the periodicity of the crystal structure by using geometrical points and lines, respectively. The interplanar spacing (the distance between two adjacent parallel crystal planes) for different crystal planes is different; the unit is Å or Angstrom.

As used herein, "angle of repose" refers to the maximum angle measured, under a gravitational field, when the equilibrium between the gravity and interparticle friction of the particles is reached as particles slide on the free slope of a powder heap and the particles come to a static state. The angle of repose of the particles is determined by using a funnel method, the particulate material flows out from the funnel and falls onto a plane to form a cone, and the angle at the base of the cone is the angle of repose. The calculation formula is as follows: $θ = {tan}^{- 1} \frac{h}{r}$ where h and r are the height and radius of the cone, respectively.

As used herein, "Carr index" refers to the compressibility index (CI), which is used to evaluate the flowability of a material. The more tightly the material is pressed in the tap density test, the poorer the flowability. The compressibility index is one of the important indexes of the flowability of powders, and its value reflects the degree of aggregation and looseness of the powders. A certain amount (W) of the powder is gently loaded into a measuring cylinder, and then the initial volume (V0) is measured. The powder is brought into a compact state by tapping, and then the final volume (Vf) is measured. The loosest bulk density (p0) and the tap density (pf) are calculated by dividing the mass W of the powder by V0 and Vf, respectively. The compressibility index CI can be calculated according to the following formula. $CI = \frac{{ρ}_{f} - {ρ}_{0}}{{ρ}_{f}}$

As used herein, "Hausner ratio (HR)" is similar to the compressibility index, and can be calculated according to the following formula. The higher the Hausner ratio is, the worse the flowability is. $HR = \frac{{ρ}_{f}}{{ρ}_{0}}$

The relationship between Carr index, Hausner ratio, and flowability is shown in the table below.

| Carr index (%) | Hausner ratio | Powder flowability |
|---|---|---|
| ≤10 | 1.00~1.11 | Excellent |
| 11~15 | 1.12~1.18 | Good |
| 16~20 | 1.19~1.25 | Fair |
| 21~25 | 1.26~1.34 | Moderate |
| 26~31 | 1.35~1.45 | Poor |
| 32~39 | 1.46~1.59 | Very poor |
| >40 | >1.60 | Extremely poor |

"Optional" or "optionally" means that the event or circumstance subsequently described may, but does not necessarily, occur, and this description includes instances where the event or circumstance occurs or does not occur. For example, "a heterocyclyl group optionally substituted with alkyl" means that alkyl may, but does not necessarily, exist; this description includes an instance where the heterocyclyl group is substituted with alkyl and an instance where it is not.

The numerical values in the present disclosure have a certain degree of error. Generally, ±10% is considered a reasonable margin of error. It is certainly necessary to consider the context in which the value is used. For example, for the content of the total impurity, the error range of the value after the measurement shall not exceed ±10%, and may be ±9%, ±8%, ±7%, ±6%, ±5%, ±4%, ±3%, ±2% or ±1%, preferably ±5%.

As used herein, "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 is an XRPD pattern of a crystal of the fumarate of the compound of formula (I) prepared in Example 1;
FIG. 2 is an XRPD pattern of a crystal of the fumarate of the compound of formula (I) prepared in Example 3;

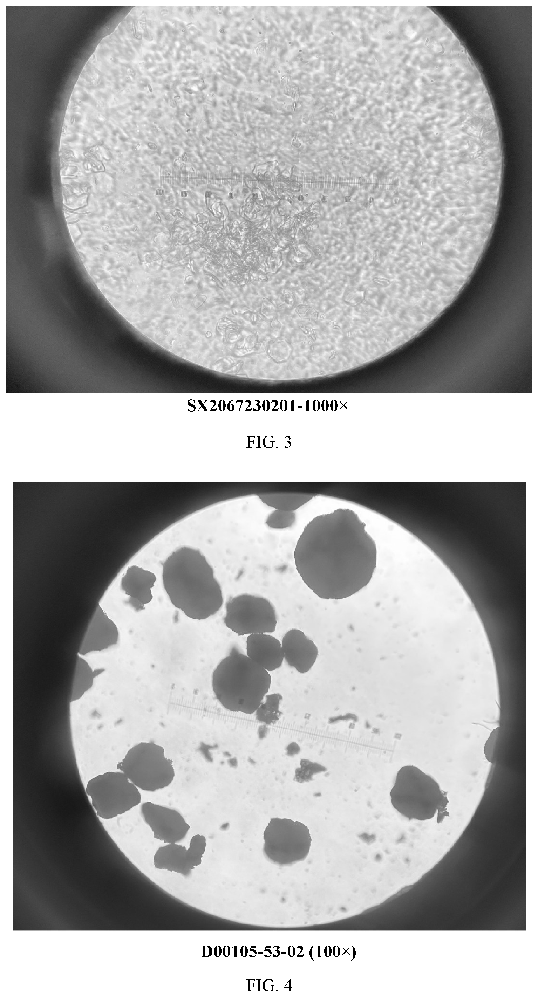

FIG. 3 is a micrograph of the crystal of the fumarate of the compound of formula (I) prepared in Example 1;
FIG. 4 is a micrograph of the crystal of the fumarate of the compound of formula (I) prepared in Example 3;

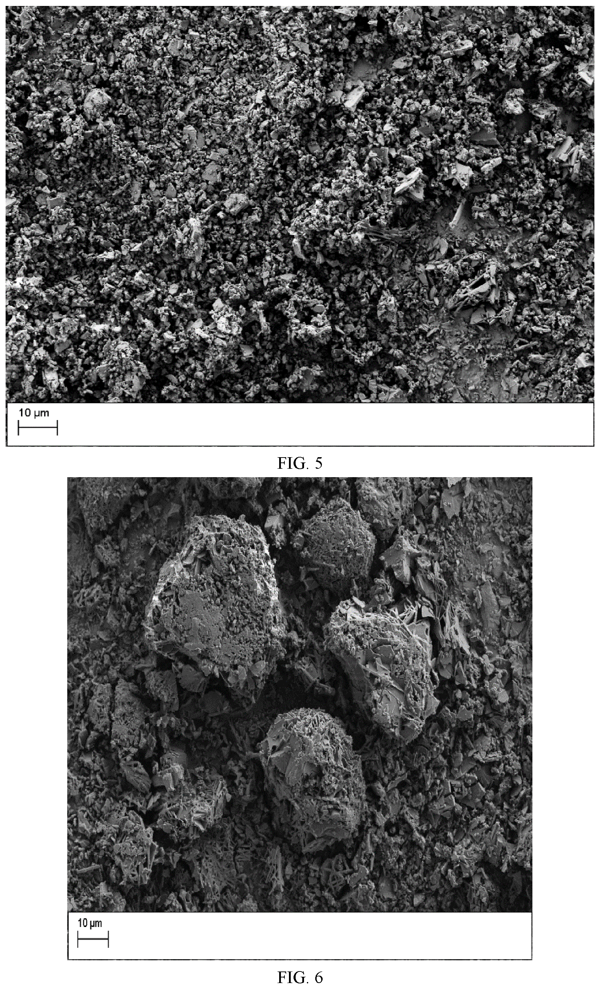

FIG. 5 is a scanning electron micrograph of the crystal of the fumarate of the compound of formula (I) prepared in Example 1; and
FIG. 6 is a scanning electron micrograph of the crystal of the fumarate of the compound of formula (I) prepared in Example 3.

### DETAILED DESCRIPTION

The present disclosure will be explained in detail below with reference to specific examples, so that those skilled in the art will more fully understand that the specific examples of the present disclosure are merely illustrative of the technical solutions of the present disclosure and do not limit the present disclosure in any way.

The compound of formula (I) described herein can be prepared according to the method in WO2022143845.

XRPD refers to X-ray powder diffraction analysis. The analysis was performed using a BRUKER D8 X-ray diffractometer, and specific acquisition information is as follows: a Cu anode (40 kV, 40 mA); ray: monochromatic Cu-Ka ray (l = 1.5418 Å). Scan mode was θ/2θ, and scan range was 3°-48°.

### Example 1

About 8 mg of compound of formula (I) and about 2.27 mg of fumaric acid were weighed out, and 0.2 mL of acetonitrile was added. The mixture was stirred for crystallization and centrifuged, and the resulting solid was dried *in vacuo* to give a product.

According to X-ray powder diffraction analysis, the product was defined as a crystal form I of fumarate. Its XRPD pattern is shown in FIG. 1, and its characteristic peak positions are shown in Table 1. The fumarate ion content was 20.34%, as determined by ion chromatography. Additional properties of the crystal form I of the fumarate are described in PCT/CN2023/104908, which is incorporated herein by reference in its entirety.

**Table 1. Peak positions of the crystal form I of the fumarate**

| Peak No. | 2*θ* value [° or degrees] | D[Å] | Relative intensity% |
|---|---|---|---|
| 1 | 4.898 | 18.02822 | 7.7 |
| 2 | 6.057 | 14.58063 | 31.1 |
| 3 | 9.634 | 9.17322 | 72.7 |
| 4 | 10.031 | 8.81136 | 62.2 |
| 5 | 10.838 | 8.15644 | 36.4 |
| 6 | 12.067 | 7.32867 | 5.1 |
| 7 | 13.275 | 6.66409 | 10.1 |
| 8 | 14.003 | 6.31931 | 51.8 |
| 9 | 14.781 | 5.98853 | 8.9 |
| 10 | 16.733 | 5.29395 | 62.1 |
| 11 | 17.225 | 5.14381 | 55.8 |
| 12 | 18.635 | 4.75775 | 40.0 |
| 13 | 19.119 | 4.63834 | 59.5 |
| 14 | 19.612 | 4.52276 | 76.2 |
| 15 | 20.212 | 4.38986 | 33.0 |
| 16 | 21.415 | 4.14603 | 5.1 |
| 17 | 21.965 | 4.04345 | 20.1 |
| 18 | 22.699 | 3.91418 | 24.6 |
| 19 | 24.171 | 3.67905 | 10.1 |
| 20 | 24.648 | 3.60902 | 6.4 |
| 21 | 25.799 | 3.45048 | 100.0 |
| 22 | 26.137 | 3.40663 | 93.3 |
| 23 | 26.639 | 3.34354 | 18.1 |
| 24 | 27.351 | 3.25814 | 7.6 |
| 25 | 28.092 | 3.17390 | 5.2 |
| 26 | 29.149 | 3.06117 | 9.1 |
| 27 | 30.041 | 2.97225 | 10.2 |
| 28 | 31.219 | 2.86269 | 4.7 |
| 29 | 31.852 | 2.80727 | 1.9 |
| 30 | 32.449 | 2.75693 | 1.6 |
| 31 | 34.523 | 2.59595 | 4.8 |
| 32 | 35.364 | 2.53613 | 10.0 |
| 33 | 36.737 | 2.44443 | 3.1 |
| 34 | 37.545 | 2.39364 | 2.6 |
| 35 | 39.197 | 2.29649 | 2.1 |
| 36 | 40.743 | 2.21283 | 2.1 |
| 37 | 41.797 | 2.15942 | 4.9 |
| 38 | 43.062 | 2.09887 | 5.1 |

### Example 2

About 80 mg of compound of formula (I) was weighed out and added to a solution of fumaric acid in methanol (0.33 mol/L, 537 µL), and 1.5 mL of acetonitrile was added. The mixture was stirred for crystallization and centrifuged, and the resulting solid was dried *in vacuo* to give a product.

According to X-ray powder diffraction analysis, the product was a crystal form I of fumarate.

### Example 3

Methanol (2100 mL, 6 V), fumaric acid (0.1 eq.), and the crystal form I of the fumarate of the compound of formula (I) prepared in Example 1 (350 g, 1.0 eq.) were added to a reaction flask, and the mixture was heated to 35 °C until complete dissolution was achieved. The resulting solution was filtered while hot, and 3-4 V of solvent was concentrated, followed by the addition of a seed crystal. Acetone (10 V) was added dropwise in portions, and the mixture was stirred. The mixture was slowly cooled to 5-10 °C, stirred for crystallization, filtered, and dried to give a crystal of the fumaric acid of the compound of formula (I). According to X-ray powder diffraction analysis, the crystal was a crystal form I of fumarate, and its XRPD pattern is shown in FIG. 2.

### Example 4

A testing device for angle of repose was set up according to the provisions of the National Standard GB11986-89 "Surface active agents-Powders and Granules-Measurement of the angle of repose" (the angle of repose of powders and particles may be measured with reference to this standard). Three parallel tests were performed. The angle of repose of the crystal of Example 3 was measured. However, the angle of repose of the crystals of Examples 1 and 2 was not determined due to their poor flowability.

In addition, the Carr index and the Hausner ratio were also measured for the crystal of Example 3, and the bulk density and tap density were also measured for the crystal of Example 3. All results are shown in Table 2.

**Table 2**

| Item | Example 1 | Example 3 |
|---|---|---|
| Crystal form | Crystal form I | Crystal form I |
| Bulk density (g/mL) | / | 0.548 |
| Tap density (g/mL) | / | 0.652 |
| Carr index (%) | >32 | 16 |
| Hausner ratio | >1.45 | 1.19 |
| Angle of repose (°) | >45 | 30.6 |

The results show that the crystal of the fumarate of the compound of formula (I) prepared in the present disclosure exhibits better flowability and is more conducive to the weighing of starting materials and the preparation of formulations. In addition, due to the good flowability and spherical-like appearance of the crystal, the crystal is dissolved more rapidly, thereby greatly improving the production efficiency.

### Example 5. Study on Stability of Crystal Form I of Fumarate in the Presence of Influencing Factors

The crystal form I of the fumarate prepared in Example 3 was spread out and left to stand in open flasks, and the samples were subjected to 30-day stability tests under light exposure (4500 Lux), high temperature (40 °C and 60 °C), and high humidity (RH 75% and RH 92.5%) conditions.

**Table 3**

| Condition | Time (days) | Color and appearance | Purity (%) | Salt form |
|---|---|---|---|---|
| Initial | 0 | White solid | 100.0 | Crystal form I of fumarate |
| Light exposure (4500 Lux) | 5 | White solid | 100.0 | Did not change |
| | 10 | White solid | 99.9 | Did not change |
| | 30 | White solid | 99.5 | Did not change |
| 40 °C | 5 | White solid | 99.7 | Did not change |
| | 10 | White solid | 99.6 | Did not change |
| | 30 | White solid | 99.3 | Did not change |
| 60 °C | 5 | White solid | 99.8 | Did not change |
| | 10 | White solid | 99.7 | Did not change |
| | 30 | White solid | 99.5 | Did not change |
| 75% RH | 5 | White solid | 100.0 | Did not change |
| | 10 | White solid | 99.9 | Did not change |
| | 30 | White solid | 100.0 | Did not change |
| 92.5% RH | 5 | White solid | 99.9 | Did not change |
| | 10 | White solid | 99.9 | Did not change |
| | 30 | White solid | 99.9 | Did not change |

Conclusion: When left to stand under light exposure, high temperature (40 °C and 60 °C), and high humidity (75% and 92.5%) conditions for 30 days, the crystal form I of the fumarate exhibited good physical and chemical stability.

### Example 6. Study on Long-Term Accelerated Stability of Crystal Form I of Fumarate

The crystal form I of the fumarate prepared in Example 3 was sealed and left to stand under 25 °C/60% RH and 40 °C/75% RH conditions to test its stability.

**Table 4**

| Test conditions | Time (months) | Purity (%) | Salt form |
|---|---|---|---|
| Initial | | 100.0 | Crystal form I of fumarate |
| 25 °C, 60% RH | 1 | 99.9 | Did not change |
| | 2 | 100.0 | Did not change |
| | 3 | 100.0 | Did not change |
| | 6 | 99.9 | Did not change |
| 40 °C, 75% RH | 1 | 99.9 | Did not change |
| | 2 | 99.9 | Did not change |
| | 3 | 99.9 | Did not change |
| | 6 | 99.8 | Did not change |

Conclusion: The long-term accelerated experiments show that when left to stand under 25 °C/60% RH and 40 °C/75% RH conditions for 6 months, the crystal form I of the fumarate exhibited good physical and chemical stability.

Since the present disclosure has been described according to special embodiments thereof, certain modifications and equivalent variations will be apparent to those skilled in the art and are included within the scope of the present disclosure.

## Claims

1. A crystal of fumarate of a compound of formula (I), wherein the crystal has an angle of repose θ of not greater than 45°, preferably not greater than 40°, and more preferably not greater than 35°,

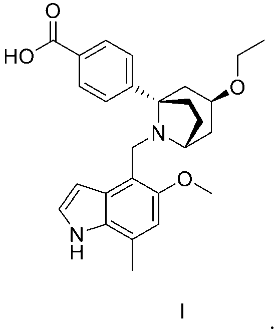

2. The crystal according to claim 1, wherein the crystal has a Hausner ratio of not greater than 1.35, preferably not greater than 1.3, and more preferably 1-1.3.

3. The crystal according to claim 1 or 2, wherein the crystal has a Carr index of not greater than 26%, preferably not greater than 21%.

4. The crystal according to any one of claims 1-3, wherein an X-ray powder diffraction pattern of the crystal, expressed in terms of 2θ angles of diffraction, shows characteristic peaks at 9.6, 14.0, 16.7, 19.6, 25.8, and 26.1, preferably at 6.1, 9.6, 10.0, 14.0, 16.7, 17.2, 19.1, 19.6, 25.8, and 26.1, and more preferably at 6.1, 9.6, 10.0, 10.8, 14.0, 16.7, 17.2, 18.6, 19.1, 19.6, 20.2, 25.8, and 26.1; most preferably, the X-ray powder diffraction pattern expressed in terms of 2θ angles of diffraction is as shown in FIG. 2.

5. The crystal according to claim 4, wherein the 2θ angles have a margin of error of ±0.2.

6. The crystal according to any one of claims 1-5, wherein the fumarate of the compound of formula (I) is in crystal form I.

7. A crystal of fumarate of a compound of formula (I), wherein the crystal has an angle of repose θ of not greater than 45°, preferably not greater than 35°; the crystal has a Hausner ratio of not greater than 1.35, preferably not greater than 1.26; the crystal has a Carr index of not greater than 26%, preferably not greater than 21%; preferably, the fumarate of the compound of formula (I) is in crystal form I.

8. A method for preparing the crystal of the fumarate of the compound of formula (I) according to any one of claims 1-7, comprising: mixing the fumarate of the compound of formula (I) with fumaric acid and a solvent under stirring to precipitate the crystal of the fumarate of the compound of formula (I), wherein the solvent is capable of being one or more of C₁-C₆ alkyl alcohol and acetone.

9. The method according to claim 8, wherein the fumarate of the compound of formula (I) and fumaric acid are first dissolved in a solvent A, followed by mixing with a solvent B to precipitate the crystal of the fumarate of the compound of formula (I), wherein the solvent A is C₁-C₆ alkyl alcohol, and the solvent B is acetone; preferably, the fumarate of the compound of formula (I) and fumaric acid are first dissolved in a solvent A, the solvent is then concentrated, optionally a seed crystal is added, and then a solvent B is added into a resulting system to precipitate the crystal of the fumarate of the compound of formula (I), wherein the solvent A is C₁-C₆ alkyl alcohol, and the solvent B is acetone.

10. The method according to claim 8 or 9, wherein a reaction temperature of the method is 0-60 °C, preferably 0-40 °C.

11. A pharmaceutical composition, comprising the crystal of the fumarate of the compound of formula (I) according to any one of claims 1-7 and one or more pharmaceutically acceptable excipients.

12. A pharmaceutical composition, wherein the pharmaceutical composition is prepared by mixing the crystal of the fumarate of the compound of formula (I) according to any one of claims 1-7 with one or more pharmaceutically acceptable excipients.

13. A method for preparing a pharmaceutical composition, comprising mixing the crystal of the fumarate of the compound of formula (I) according to any one of claims 1-7 with one or more pharmaceutically acceptable excipients.

14. A drug substance of fumarate of a compound of formula (I), comprising the crystal of the fumarate of the compound of formula (I) according to any one of claims 1-7.

15. Use of the crystal of the fumarate of the compound of formula (I) according to any one of claims 1-7, the drug substance according to claim 14, or the pharmaceutical composition according to claim 12 or 13 in the manufacture of a medicament for treating a disease or disorder, wherein the disease or disorder is preferably glomerulopathy, hemolytic uremic syndrome, atypical hemolytic uremic syndrome, paroxysmal nocturnal hemoglobinuria, age-related macular degeneration, geographic atrophy, diabetic retinopathy, uveitis, retinitis pigmentosa, macular edema, Behcet's uveitis, multifocal choroiditis, Vogt-Koyanagi-Harada syndrome, birdshot retino-choroiditis, sympathetic ophthalmia, ocular cicatricial pemphigoid, ocular pemphigus, nonarteritic ischemic optic neuropathy, post-operative inflammation, retinal vein occlusion, neurological disorders, multiple sclerosis, stroke, Guillain-Barré syndrome, traumatic brain injury, Parkinson's disease, disorders of inappropriate or undesirable complement activation, hemodialysis complications, hyperacute allograft rejection, xenograft rejection, interleukin-2 induced toxicity during IL-2 therapy, Crohn's disease, adult respiratory distress syndrome, myocarditis, post-ischemic reperfusion conditions, myocardial infarction, balloon angioplasty, post-pump syndrome in cardiopulmonary bypass or renal bypass, atherosclerosis, hemodialysis, renal ischemia, mesenteric artery reperfusion after aortic reconstruction, infectious diseases or sepsis, systemic lupus erythematosus, systemic lupus erythematosus nephritis, proliferative nephritis, liver fibrosis, hemolytic anemia, myasthenia gravis, tissue regeneration, neural regeneration, dyspnea, hemoptysis, acute respiratory distress syndrome, asthma, chronic obstructive pulmonary disease, emphysema, pulmonary embolisms and infarcts, pneumonia, fibrogenic dust diseases, pulmonary fibrosis, asthma, allergy, bronchoconstriction, parasitic disease, Goodpasture's syndrome, pulmonary vasculitis, pauci-immune vasculitis, immune complex-associated inflammation, antiphospholipid syndrome, and obesity; the disease or disorder is more preferably C3 glomerulopathy, IgA nephropathy, membranous glomerulonephritis, atypical hemolytic uremic syndrome, and paroxysmal nocturnal hemoglobinuria.
